# EUROPEAN PATENT APPLICATION

(11) **EP 4 696 371 A1**
(43) Date of publication of application: **18.02.2026**
(21) Application number: 23936717.0
(22) Date of filing: 29.11.2023
(51) Int. Cl.: A61N 1/40, A61N 1/36, A61N 1/32, A61N 1/05, A61M 5/32

(54) **SKIN SURGERY DEVICE EQUIPPED WITH NEEDLE GROUP AND DRIVING METHOD THEREOF**

(30) Priority: 11.05.2023 KR 20230061232
(71) Applicant: Piamolifting.Co.,Ltd, Gyeongsangbuk-do 38577 (KR)
(72) Inventor: PARK, Won Hee, Seoul 06715 (KR)
(74) Representative: Flügel Preissner Schober Seidel
(86) International application number: PCT/KR2023/019447
(87) International publication number: WO 2024/232494

(57) **Abstract**

A skin surgery device equipped with a needle group and a driving method thereof are disclosed. The skin surgery device equipped with a needle group, according to an embodiment of the present invention, comprises: a needle group comprising a plurality of needles; a first circuit board to which a first needle group that is a part of the needle group is fixed and in which a perforated portion is formed; and a second circuit board to which a second needle group that is the other part of the needle group is fixed, wherein the second needle group is formed to pass through and protrude out of the perforated portion of the first circuit board.

## Description

### Technical Field

The present disclosure relates to a device that is effective in skin beauty or lesion treatment, such as promoting the decomposition of skin fat tissue in a treatment area or forming collagen by forming a wound on the skin surface of a treatment area through a needle and then irradiating radio frequency (RF) high-frequency energy through the needle, and more specifically, to a device that may further improve the therapeutic effect by densely disposing needles at intervals in a limited space area, and a method of driving the same.

### Background Art

Korean Patent No. 10-1300122 discloses a needle for high-frequency treatment comprising a plurality of needles, a support plate supporting the plurality of needles on one surface, a base supporting a back surface of the support plate, a cover coupled to the base and having a penetration hole formed in one side wall facing one surface of the support plate through which the plurality of needles penetrate, and an elastic body configured to elastically support the support plate from the one side wall of the cover, wherein the elastic body comprises a first elastic part configured to elastically support a first region, which is a portion of an edge part of the support plate, and a second elastic part configured to elastically support a second region, which is an edge part region of the support plate excluding the first region, wherein the plurality of needles are configured to be sequentially inserted into skin according to elasticity or shape of the first elastic part and the second elastic part.

In addition, Korean Patent No. 10-2117711 discloses an RF high-frequency device comprising a needle part which is inserted into a dermis layer to emit RF high-frequency energy, a needle insert part in which at least one or more of the needle parts are inserted by penetrating in a row and are installed by being sequentially connected in a front-back direction to form a bundle, and a printed circuit board (PCB) part installed on an upper side of the needle insert part bundle installed by being connected to each other to support the needle part and configured to control RF high-frequency energy emission of the needle part.

In addition, Korean Patent No. 10-2151923 discloses a needle laser treatment system comprising an optical cable for providing laser generated from a laser generator, a handpiece having multiple lenses installed inside for amplifying or expanding the laser received from the optical cable, and a needle unit connected to a lower end of the handpiece and configured to receive the laser through the handpiece and directly irradiate the laser to a treatment area within skin by passing through skin surface, wherein the handpiece comprises a focus lens into which the laser provided from the optical cable is incident, a convex lens for expanding or amplifying the laser emitted from the focus lens, a collimating lens for emitting the laser emitted from the convex lens in a direction parallel to a propagation direction, and an optical lens part connected to the needle unit and having a plurality of optical lenses installed to expand or amplify the laser emitted from the collimating lens and transmit the laser to the needle unit.

However, the needle unit configured in the conventional skin treatment devices has a problem in that a relatively wide area is formed between the needles (e.g., a rectangle formed by four adjacent needles) due to the disposition structure of the needles that are connected to the electric circuit of the PCB board to supply high-frequency energy and fixed to a separate support plate, but the needles cannot penetrate into this area.

In addition, there is a problem in that there is a limit to densely disposing the intervals of needles in a limited area due to the diameter of the needle fixing part for fixing the needles, and as a result, there is a problem that the treatment effect may be reduced due to the far distance between the needles when the needles are arranged.

### [Prior-Art Documents]

### - Patent Documents:

Korean Patent No. 10-2384711
Korean Patent No. 10-2117711
Korean Patent No. 10-2151923

### Disclosure of the Invention

### Technical Goals

The present disclosure has been devised to address the above problems, and an object of the present disclosure is to provide a technical idea that allows needles to be densely disposed in a limited cross-sectional area without the risk of short circuits.

Another object of the present disclosure is to provide a device that improves safety and convenience of treatment, so as to effectively treat skin or effectively decompose and remove subcutaneous fat tissue.

### Technical Solutions

A skin treatment device provided with a needle group according to an embodiment of the present disclosure for solving the above technical problem includes a needle group including a plurality of needles; a first circuit board in which a first needle group, which is a part of the needle group, is fixed and a perforation part is formed; and a second circuit board in which a second needle group, which is the remaining part of the needle group, is fixed, wherein the second needle group is formed to protrude outward through the perforation part of the first circuit board.

The skin treatment device may further include a tip case provided with a needle penetration hole through which the first needle group and the second needle group penetrate and protrude outward.

The number of needles included in the first needle group may be greater than the number of needles included in the second needle group.

First needle fixing parts corresponding to the first needle group may be arranged in a matrix having a plurality of rows and a plurality of columns, and the perforation part may be formed in a free space inside an area formed by the matrix that does not overlap with the first needle fixing parts.

The perforation part may be formed inside a minimum rectangle or a minimum triangle that needle fixing parts included in the matrix are able to form.

Needles included in the second needle group may be formed to have a greater length than needles included in the first needle group.

The first needle group and the second needle group may be formed such that respective end points are positioned at the same depth when maximally protruded outward.

At least one of the needles included in the needle group may be provided with a hollow part to enable drug supply.

The skin treatment device may further include a needle circuit board which is simultaneously connected to the first circuit board and the second circuit board so as to supply RF high-frequency energy to needles included in the first needle group and the second needle group, and a bracket configured between the second circuit board and the needle circuit board to support the needle circuit board.

The skin treatment device may be configured such that an elastic spring having one end supported by the needle circuit board and a needle electrode rod fitted into the elastic spring are interposed such that circuit connections are made between the needle circuit board and the first circuit board and the second circuit board.

The skin treatment device may include a main circuit board connected to the needle circuit board and a vibration motor, and further include a needle moving means configured to push at least the first circuit board and the second circuit board such that the needles included in the first needle group and the second needle group pass through the needle penetration hole of the tip case.

A skin treatment device provided with a needle group according to another aspect may include a first needle group; and a second needle group, wherein the first needle group may be arranged in a matrix having a plurality of rows and a plurality of columns, and the second needle group may be formed inside a minimum rectangle or a minimum triangle that needles included in the matrix are able to form.

According to another aspect of the present disclosure, in a skin treatment device including a plurality of needles, the plurality of needles include a first needle group arranged in a rectangular matrix of m×n (where m is a natural number greater than or equal to 2 and the number of rows, and n is a natural number greater than or equal to 2 and the number of columns), and a second needle group disposed inside a minimum rectangle formed by a needle disposed at (a, b) (where a is a row greater than or equal to 1 and less than or equal to (m-1), and b is a column greater than or equal to 1 and less than or equal to (n-1)), a needle disposed at (a, b+1), a needle disposed at (a+1, b), and a needle disposed at (a+1, b+1), among the first needle group.

According to another aspect of the present disclosure, in a skin treatment device including a plurality of needles, the plurality of needles include a first needle group arranged in a plurality of m rows (m is a natural number greater than or equal to 2), wherein among the m rows, n (n is a natural number greater than or equal to 2) needles are disposed in any one row a (a is a natural number greater than or equal to 1 and less than or equal to (m-1)) and (n-1) needles are disposed in row (a+1), wherein the needles disposed in the row (a+1) are disposed so as to be positioned between the needles of the row a in an x-axis direction, and a second needle group disposed inside (e.g., at the center of) a minimum triangle formed by a needle disposed at (a, b) (a is a natural number greater than or equal to 1 and less than or equal to (m-1), and b is a natural number greater than or equal to 1 and less than or equal to (n-1)), a needle disposed at (a, b+1), and a needle disposed at (a+ 1, b), among the first needle group.

A method of driving a skin treatment device provided with a needle group according to the technical idea of the present disclosure may include, receiving, by the skin treatment device provided with the needle group, a treatment start signal, and moving, by the skin treatment device provided with a needle group, a first circuit board in which a first needle group, which is a part of a needle group including a plurality of needles, is fixed and a perforation part is formed, and a second circuit board in which a second needle group, which is the remaining part of the needle group, is fixed, in response to the treatment start signal, wherein the second needle group may be formed to protrude outward through the perforation part of the first circuit board.

### Advantageous Effects

According to the technical idea of the present disclosure, there is an effect of, when a plurality of needles are arranged to penetrate or stimulate the skin, reducing the area that the needles cannot reach, thereby disposing the intervals of needles relatively densely.

In particular, by fixing some of the plurality of needles to a first circuit board and fixing the remaining some to a second circuit board, the needles may be disposed relatively densely, and there is an effect that, even when the needles are disposed densely in a limited space, the occurrence of short circuit defects when soldering and fixing the needles to the circuit board may be prevented.

In addition, since the needles may be disposed densely, there is an advantage in that the treatment area may be focused and treated effectively.

### Brief Description of Drawings

In order to more fully understand the drawings cited in the detailed description of the present disclosure, a brief description of each drawing is provided.
FIG. 1 is a three-dimensional view of a skin treatment device provided with a needle group of the present disclosure.
FIG. 2 is a three-dimensional view illustrating a state of a skin treatment device provided with a needle group of the present disclosure separated from a body part.
FIG. 3 is an exploded perspective view of a skin treatment device provided with a needle group of the present disclosure.
FIG. 4 is a cross-sectional view of a skin treatment device provided with a needle group of the present disclosure.
FIG. 5 is an exploded perspective view of a body block configured in a body part of a skin treatment device provided with a needle group of the present disclosure.
FIG. 6 is an exploded perspective view of a portion of a skin treatment device provided with a needle group of the present disclosure.
FIGS. 7A and 7B are plan views illustrating a needle fixing part of a first circuit board of the present disclosure.
FIG. 8 is a plan view illustrating a needle fixing part of a second circuit board of the present disclosure.
FIG. 9 is an enlarged three-dimensional view illustrating a tip case of the present disclosure.
FIG. 10 is a diagram for explaining a conventional needle disposition method.
FIG. 11 is a diagram for explaining a needle disposition shape according to an embodiment of the present disclosure.

### Best Mode for Carrying Out the Invention

Since the present disclosure may apply various transformations and have various embodiments, specific embodiments will be illustrated in the drawings and described in detail in the detailed description. However, this is not intended to limit the present disclosure to specific embodiments, and it should be understood to include all transformations, equivalents and substitutes included in the spirit and scope of the present disclosure. In describing the present invention, if it is determined that a detailed description of related known technologies may obscure the gist of the present disclosure, the detailed description will be omitted.

Terms such as first and second may be used to describe various components, but the components should not be limited by the terms. Such terms are used only for the purpose of distinguishing one component from another.

The terms used in the present application are used only to describe a particular embodiment and are not intended to limit the present disclosure. Singular expressions include plural expressions unless the context clearly means otherwise.

In this specification, it should be understood that terms such as "comprise", "include" or "have" are intended to designate the presence of features, numbers, steps, operations, components, parts, or combinations thereof described in the specification, and do not preclude the possibility of the presence or addition of one or more other features, numbers, steps, operations, components, parts, or combinations thereof.

Hereinafter, with reference to the accompanying drawings, the present disclosure will be described in detail centering on embodiments of the present disclosure. Like reference numerals in each figure indicate like members.

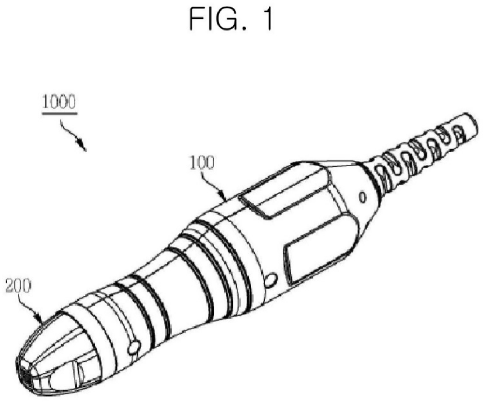

A skin treatment device 1000 provided with a needle group of the present disclosure may include a body part 100 that also functions as a handle of a handpiece, as shown in FIGS. 1 to 3, and a needle unit 200 detachably coupled to the body part.

The skin treatment device 1000 provided with a needle group may be a device used for skin beauty or skin treatment by penetrating a plurality of needles into the skin and then irradiating electric energy thereon. Depending on the embodiment, the skin treatment device 1000 provided with a needle group may be a device used for the purpose of providing only a certain level of stimulation from the outside of the skin without penetrating the skin to form a wound.

The body part 100 includes a motor block 300 mounted on the inner upper side of a body case 501, 502, and a body block 400 mounted on the inner lower side of the body case 501, 502. The RF high-frequency energy and electric energy supplied to the skin treatment device 1000 are supplied through a cable 520 coupled to the upper side of the body case 501, 502, and for the convenience of explanation, the connection of the circuit wiring is omitted. The motor block 300 may include a driving motor 301 and a shaft 302 that advances and retreats along the central axis direction by the power of the driving motor.

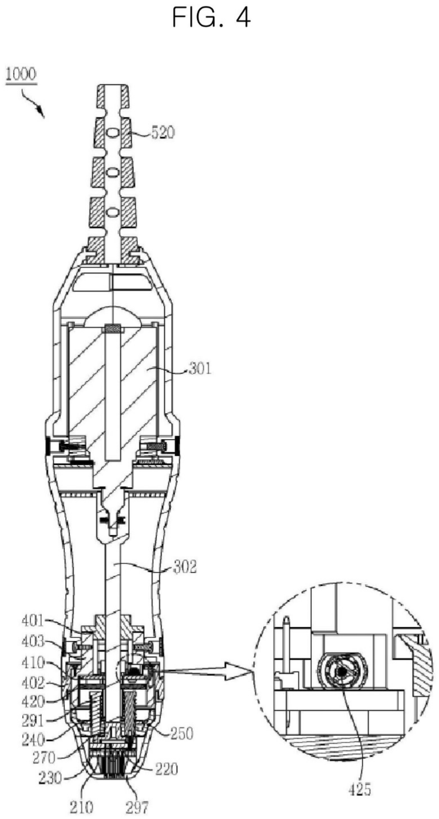

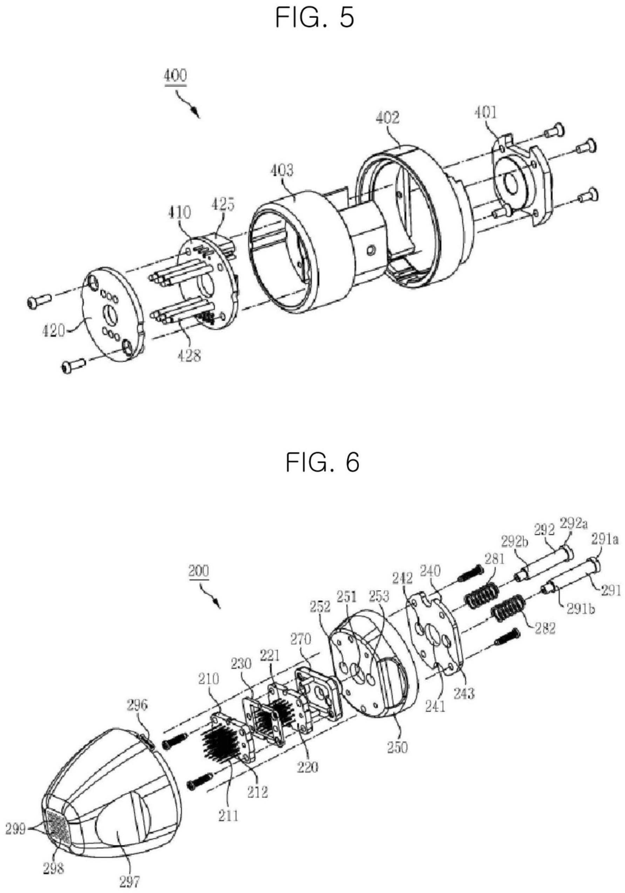

Next, the technical configuration and operation of the body block 400 of the present disclosure will be described mainly with reference to FIGS. 4 and 5.

The body block 400 may include a shaft support part 401, a front support part 402, a main circuit board support part 403, a main circuit board 410, and a light emitting diode (LED) window 420.

The front support part 402 is coupled to the shaft support part 401 and the main circuit board support part 403 and is fixed to the lower end part of the body case 501, 502. The main circuit board 410 and the LED window 420 are connected to the main circuit board support part 403.

The main circuit board 410 may be configured with a circuit to supply RF high-frequency energy to the needle. In addition, the main circuit board 410 may be configured with a connection pin 428 that is electrically connected to the circuit board to which the needle is fixed when the needle unit 200 is connected to the body part 100, and may include a vibration motor 425 that causes vibration in the body part 100.

The vibration motor 425 may play a role in alleviating the stiffness of the skin by gently massaging the stiffened skin in advance when a wound is formed on the skin of the treatment area with the needle. In addition, it may also provide an effect of reducing fatigue when the subject of the treatment holds the body part 100 for a long time during treatment.

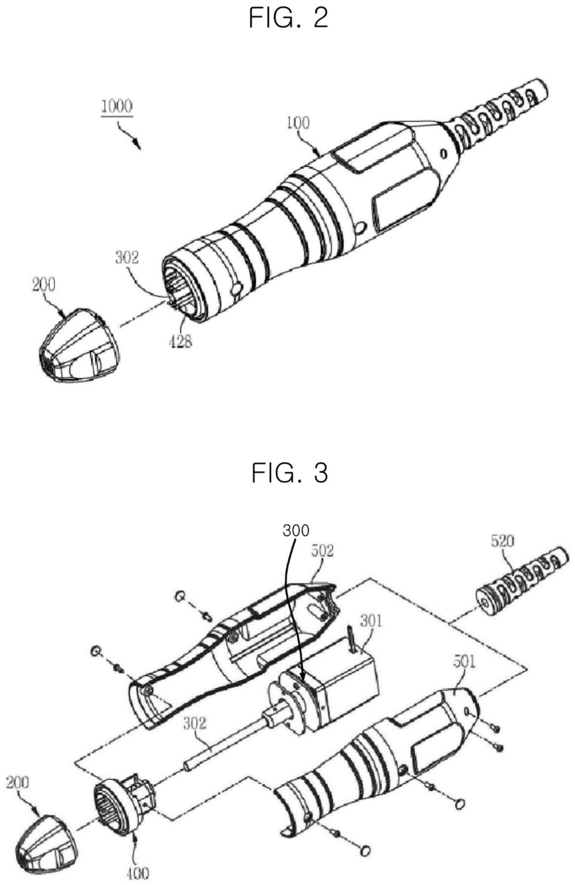

The LED window 420 serves as a display window to indicate the driving status of the treatment device during the treatment process. The body block 400 configured as described above may be configured so that the end part of the shaft 302 and the connection pin 428 of the main circuit board are exposed by penetrating the LED window 420, as shown in FIG. 2.

In addition, the needle unit 200 of the present disclosure may include a needle group 211, 221 including a plurality of needles.

A first needle group 211, which is a part of the needle group 211, 221 may be fixed to the first circuit board 210. In addition, the second needle group 221, which is the remaining part, may be fixed to the second circuit board 220. The second needle group 221 is disposed so as to be able to penetrate a perforation part 212 formed in the first circuit board 210 and protrude outward, thereby being able to penetrate the skin.

In other words, according to the technical idea of the present disclosure, by fixing and disposing the plurality of needles to different circuit boards, it is possible to solve a problem that may occur when disposing many needles to a single circuit board.

For example, as shown in FIGS. 4 and 6, the needles included in the first needle group 211 are fixed, and a first circuit board 210 having the perforation part 212 may be included.

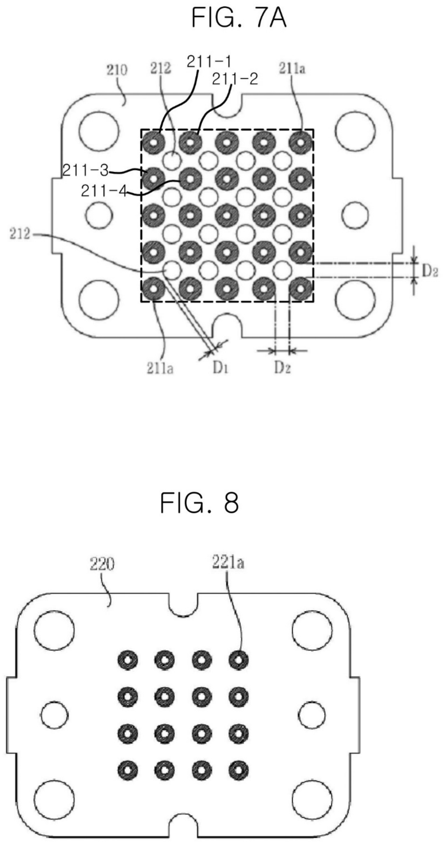

The first needle group 211 may be fixed to a needle fixing part 211a that is arranged at a regular interval on the back surface of the first circuit board 210 in a predetermined manner, as shown in FIG. 7A. For example, it may be fixed by various fixing methods such as soldering.

According to another embodiment, the circuit board and needles defined in the present specification may be formed integrally. For example, a protrusion part may be formed on a copper plate so that the protrusion part functions as a needle and the copper plate functions as a circuit board. The technical idea of the present disclosure is that the circuit board and the needles may be provided in various ways, and the average expert in the technical field of the present disclosure may easily infer that the needles being coupled or disposed on the circuit board may include a case where the circuit board and the needles are formed integrally.

The technical idea of the present disclosure is exemplified by an example in which the needles fixed to the first circuit board 210, i.e., the circuit board disposed closer to the skin side, i.e., the first needle group 211, are disposed in a predetermined matrix on the outer side, and the needles fixed to the second circuit board 220, i.e., the circuit board disposed farther from the skin side, i.e., the second needle group 221, are disposed between the matrixes, but the present disclosure is not necessarily limited thereto.

In other words, FIG. 7A exemplarily illustrates a case where the number of needles included in the first needle group 211 is greater and the number of needles included in the second needle group 221 is less, and the second needle group 221 is disposed between the interiors of the area (dotted line area in FIG. 7) where the first needle group 211 is disposed. However, depending on the embodiment, the number of needles included in the first needle group 211 fixed to the first circuit board 210 may be implemented to be less than the number of needles included in the second needle group 221 fixed to the second circuit board 220, and in this case, an average expert in the technical field of the present disclosure will be able to easily infer that the needle group 211, 221 may be implemented so that the first needle group 211 is disposed between the second needle group 221.

In addition, according to the technical idea of the present disclosure, it may be advantageous to achieve a uniform therapeutic effect if the depth of the end point reached by the first needle group 211 and the second needle group 221 penetrating the skin is all constant. The first needle group 211 and the second needle group 221 do not necessarily need to penetrate the skin, and may be used for the purpose of stimulating the skin by pressing it to a certain level. In any case, it may be preferable that the needles included in the second needle group 221 are longer than the needles included in the first needle group 211 by a certain length or more. However, the scope of the present disclosure is not necessarily limited thereto, and various embodiments may be possible.

According to an embodiment, the needles included in the first needle group 211 may be fixed to the corresponding first needle fixing parts 211a, respectively.

The first needle fixing parts 211a may be arranged in a matrix having a plurality of rows and a plurality of columns, as illustrated in FIG. 7A. However, embodiments of the arrangement of the first needle fixing parts 211a may vary.

For example, FIG. 7A illustrates a case in which the first needle fixing parts 211a are arranged in a 5×5 matrix, and exemplarily illustrates a rectangular matrix in which each row is disposed at the same position in the x-axis direction. Typically, the first needle fixing parts 211a may be arranged in an m×n rectangular matrix (where m is a natural number greater than or equal to 2 and the number of rows, and n is a natural number greater than or equal to 2 and the number of columns).

A plurality of perforation parts 212 may be formed in a free space that does not overlap with the first needle fixing parts 211a inside the area (dotted line area) formed by the matrix.

For example, the first needle fixing parts 211a may be arranged in a rectangular matrix as illustrated in FIG. 7A, and in this case, the perforation part 212 may be formed inside a minimum rectangle that is the smallest of the rectangles that may be formed by any four needle fixing parts (e.g., 211-1, 211-2, 211-3, 211-4) included in the first needle fixing parts 211a. The perforation part 212 may be positioned, for example, at the center of the minimum rectangle.

In other words, the rectangle formed by four adjacent first needle fixing parts 211a may be the minimum rectangle, and in this case, it may be preferable to place one more needle in the inner space of the minimum rectangle. However, according to the technical idea of the present disclosure, the needles to be disposed in the inner space of the minimum rectangle are not fixed to the same physical configuration as the four needle fixing parts (e.g., 211-1, 211-2, 211-3, 211-4) forming the minimum rectangle, i.e., the first circuit board 210, but are fixed to a different physical configuration, i.e., the second circuit board 220, thereby having an effect of making the interval between the needles denser and at the same time solving the short circuit problem between the needle fixing part 211a for fixing the needles.

For example, a perforation part 212 may be formed at the center of the respective minimum rectangle formed by the four needle fixing parts 211a. By forming the perforation part 212 in the center of four adjacent needle fixing parts 211a, the perforation part 212 may be positioned at a position forming an equilateral triangle with two adjacent first needles (e.g., 211-1, 211-2) of the first needle group 211.

In general, when the first needle fixing parts are arranged in a rectangular matrix of m×n (m is a natural number greater than or equal to 2 and the number of rows, and n is a natural number greater than or equal to 2 and the number of columns), the needle fixing part disposed at (a, b) (where a is a row greater than or equal to 1 and less than or equal to (m-1) and b is a column greater than or equal to 1 and less than or equal to (n-1)), the needle fixing part disposed at (a, b+1), the needle fixing part disposed at (a+1, b), and the needle fixing part disposed at (a+1, b+1) may form a minimum rectangle. For example, when a and b are 1, the needle fixing part 211-1 disposed at (1, 1), the needle fixing part 211-2 disposed at (1, 2), the needle fixing part 211-3 disposed at (2, 1), and the needle fixing part 211-4 disposed at (2, 2) may form a minimum rectangle. In this case, a perforation part (e.g., 212) may be formed inside (e.g., in the center) of the minimum rectangle.

Ultimately, since the disposition of the needles penetrating the skin becomes also the same as the disposition of the needle fixing parts, by disposing one more needle in the center of the inside of the minimum rectangle formed by four needles, there is an effect of densely disposing the interval between the needles.

Meanwhile, FIG. 7 illustrates an example in which the first needle group is arranged in a rectangular matrix, such that one row (e.g., the first row including 211-1) and the next row (e.g., the second row including 211-3) are disposed at the same position in the horizontal direction. In other words, the i^{th} needle (i is greater than or equal to 2 and less than or equal to n) included in the first row of m×n (m and n are natural numbers greater than or equal to 2) of the first needle group and the i^{th} needle included in the second row may exist at the same position in the x-axis, but may exist at positions spaced apart by a predetermined interval (D2) only in the y-axis direction. In this case, the first needle group may have four needles (e.g., 211-1, 211-2, 211-3, 211-4) forming a minimum rectangle.

However, according to another embodiment, respective needles of the first needle group included in the second row may be disposed between the needles of the first row. An example of this is illustrated in FIG. 7B.

As illustrated in FIG. 7B, the first needle fixing parts may include a plurality of m rows. Row a may have n elements, and each element may represent a position at which the first needle fixing part is disposed.

In addition, row (a+1) may have (n-1) elements, and each element of the row (a+1) may be positioned between the elements of row a in the x-axis direction, as illustrated in FIG. 7B.

In this case, the needle fixing part disposed at (a, b) (a is a natural number greater than or equal to 1 and less than or equal to (m-1) and b is a natural number greater than or equal to 1 and less than or equal to (n-1)), the needle fixing part disposed at (a, b+1), and the needle fixing part disposed at (a+1, b) may form a minimum triangle. For example, when a and b are 1, the needle fixing part 211-5 disposed at (1, 1), the needle fixing part 211-6 disposed at (1, 2), and the needle fixing part 211-4 disposed at (2, 1) may form a minimum triangle. In this case, a perforation part (e.g., 212) may be formed inside (e.g., in the center) of the minimum triangle.

Meanwhile, according to another embodiment, the skin treatment device 1000 provided with a needle group may have only one circuit board. Even in this case, the skin treatment device 1000 provided with a needle group may have the first needle group and the second needle group fixed to one circuit board, and in this case, the first needle fixing parts to which the first needle group is fixed may be arranged in a matrix having a plurality of rows and a plurality of columns, as illustrated in FIG. 7A. In addition, instead of the perforation part shown in FIG. 7A, needle fixing parts may be disposed.

The second needle fixing parts corresponding to the second needle group, which are disposed at the positions of the perforation parts, may be formed inside the minimum rectangle among the rectangles that may be formed by any four needle fixing parts (e.g., 211-1, 211-2, 211-3, 211-4) included in the first needle fixing parts. In other words, as described above, the needles included in the second needle group may be disposed inside the minimum rectangle formed by four adjacent first needle fixing parts. In this case, there is an effect that needles may be disposed relatively densely without configuring a plurality of circuit boards. In this case, the possibility of electrical shorts between the needle fixing parts may increase further.

In general, when the needle fixing parts are arranged in a rectangular matrix of m×n (m is a natural number greater than or equal to 2 and the number of rows, and n is a natural number greater than or equal to 2 and the number of columns), the needle fixing part disposed at (a, b) (where a is a row greater than or equal to 1 and less than or equal to (m-1) and b is a column greater than or equal to 1 and less than or equal to (n-1)), the needle fixing part disposed at (a, b+1), the needle fixing part disposed at (a+1, b), and the needle fixing part disposed at (a+1, b+1) may form a minimum rectangle. For example, when a and b are 1, the needle fixing part 211-1 disposed at (1, 1), the needle fixing part 211-2 disposed at (1, 2), the needle fixing part 211-3 disposed at (2, 1), and the needle fixing part 211-4 disposed at (2, 2) may form a minimum rectangle. In this case, one more needle fixing part may be formed inside (e.g., in the center) of the minimum rectangle.

Ultimately, since the disposition of the needles penetrating the skin becomes also the same as the disposition of the needle fixing parts, by disposing one more needle in the center of the inside of the minimum rectangle formed by four needles, there is an effect of densely disposing the interval between the needles.

According to another embodiment, as illustrated in FIG. 7B, the first needle fixing parts may include a plurality of m rows. Row a may have n elements, and each element may represent a position at which the first needle fixing part is disposed.

In addition, row (a+1) may have (n-1) elements, and each element of the row (a+1) may be positioned between the elements of row a in the x-axis direction, as illustrated in FIG. 7B.

In this case, the needle fixing part disposed at (a, b) (a is a natural number greater than or equal to 1 and less than or equal to (m-1) and b is a natural number greater than or equal to 1 and less than or equal to (n-1)), the needle fixing part disposed at (a, b+1), and the needle fixing part disposed at (a+1, b) may form a minimum triangle. For example, when a and b are 1, the needle fixing part 211-5 disposed at (1, 1), the needle fixing part 211-6 disposed at (1, 2), and the needle fixing part 211-4 disposed at (2, 1) may form a minimum triangle. In this case, one additional needle fixing part may be formed inside (e.g., in the center) of the minimum triangle.

Ultimately, since the disposition of the needles penetrating the skin becomes also the same as the disposition of the needle fixing parts, by disposing one more needle in the center of the inside of the minimum triangle formed by three needles, there is an effect of densely disposing the interval between the needles.

Ultimately, according to the technical idea of the present disclosure, whether the needles are fixed by dividing them into a plurality of circuit boards or fixed to a single circuit board, one more needle may be disposed inside the minimum rectangle or the rectangle triangle in the conventional needle disposition method, so that a higher skin treatment effect may be obtained.

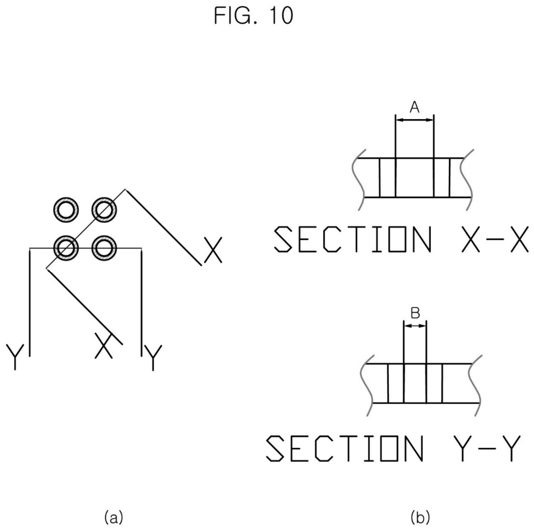

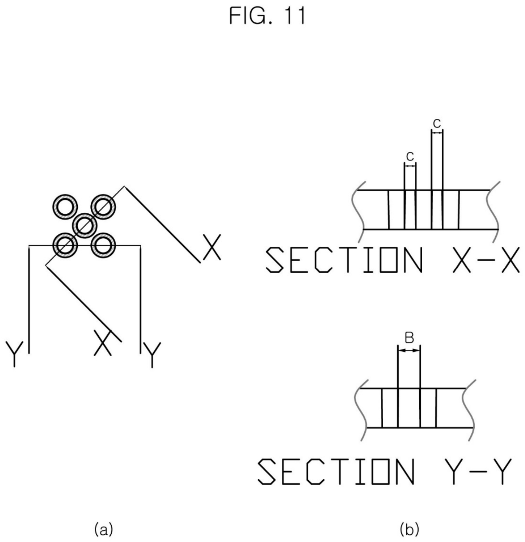

An example of this will be described in more detail with reference to FIGS. 10 and 11.

FIG. 10 is a diagram for explaining a conventional needle disposition method, and in order to fix the needle, the needle fixing part is required to occupy a certain level or more of area. Otherwise, the bonding strength between the needle and the circuit board may not be sufficient, and in this case, the needle may come off the circuit board or come out through the opposite side of the circuit board, and in cases where the needle penetrates the skin and discharges electric energy, or the skin and the needle pin may contract, causing the needle to be stuck in the skin and not come out.

Therefore, due to the size of the needle fixing part exceeding a certain level, the needle fixing part was conventionally disposed in a rectangular matrix form as illustrated in FIG. 10A.

Then, as illustrated in FIG. 10B, in the interval between needles included in the same row, i.e., Y-Y, the interval between the perforated skin, i.e., the length of the surface area of the skin that is not perforated, becomes approximately B. However, in the interval X-X between needles located in the diagonal direction, a relatively much larger interval, approximately A, occurs. For example, when B is about 1.1 mm, A becomes an interval of about 1.95 mm, which may mean that the non-perforated skin area becomes relatively larger, thereby reducing the treatment effect.

However, according to the disposition method of the needle fixing part according to the technical idea of the present disclosure as illustrated in FIG. 11, while sufficiently guaranteeing the area of the needle fixing part, much denser needle disposition is possible, thereby increasing the treatment effect.

Referring to FIG. 11, when the needle fixing part is disposed in a rectangular matrix as described above, the perforation part or the needle fixing part may be disposed so that one more needle may be disposed inside (e.g., in the center) the minimum rectangle. When the perforation part is disposed inside (e.g., in the center) the minimum rectangle, it may be a case where the needle is coupled to a plurality of circuit boards, and when the needle fixing part is disposed inside (e.g., in the center) the minimum rectangle, it may be a case where the needle is coupled to a single circuit board. In this case, the interval between needles included in the same row, i.e., the interval between the perforated skin in Y-Y is still approximately B. However, in the interval X-X between needles located in the diagonal direction, a relatively much narrower interval, approximately C, occurs. For example, when B is about 1.1 mm, C becomes an interval of about 0.58 mm, which may mean that the non-perforated skin area becomes relatively smaller, thereby greatly improving the treatment effect compared to the conventional method.

Meanwhile, as described above, when the needle fixing parts included in a specific row of the needle fixing parts are positioned between the needle fixing parts of the previous row in the horizontal direction, the perforation part or needle fixing part may be disposed so that one more needle may be disposed inside (e.g., in the center) of the minimum triangle formed by three needle fixing parts. When the perforation part is disposed inside (e.g., in the center) the minimum triangle, it may be a case where the needle is coupled to a plurality of circuit boards, and when the needle fixing part is disposed inside (e.g., in the center) the minimum triangle, it may be a case where the needle is coupled to a single circuit board. In this case, the interval between needles included in the same row, i.e., the interval between the perforated skin in Y-Y becomes approximately D. However, the interval between the needles formed along X-X becomes a relatively much narrower interval, which is an interval of approximately C. For example, when D is about 1.6 mm, C becomes an interval of about 0.58 mm, and even in this case, the non-perforated skin area becomes relatively smaller, which may mean that the treatment effect is greatly increased compared to the conventional method.

Referring again to FIG. 7, the interval D1 between any one of the needle fixing parts 211a corresponding to the first needle group 211 and the adjacent perforation part 212 may be configured as 0.28±0.1 mm, the interval D2 between the needle solder part 211a and the adjacent needle solder part 211a may be configured as 0.75±0.1 mm, the diameter of the needle solder part 211a may be configured as 1.2±0.1 mm, and the diameter of the perforation part 212 may be configured as 1±0.1 mm. Various embodiments may be possible.

In addition, the second circuit board 220 to which the second needle group 221 passing through the perforation part 212 of the first circuit board 210 is fixed may be further included in the skin treatment device 1000 provided with a needle group, and the second needle group 221 may be fixed to a second needle solder part 221a configured to be arranged at a certain interval on the back surface of the second circuit board 210 as shown in FIG. 8 in a predetermined manner. The second needle group 221 may also be fixed to the second needle solder part 221a in various ways, such as by soldering.

The diameter of each of the needles fixed to the first circuit board 210 and the second circuit board 220 as described above may be configured to be 700 µm or less. If the diameter of the needle is 700 µm or more, unnecessary pain and scarring on the skin may increase.

Ultimately, according to the technical idea of the present disclosure, by dividing and fixing the needles to a plurality of circuit boards, the needles may be densely disposed at intervals in a limited space area, and the occurrence of short-circuit defects when soldering and fixing the needles to each circuit board may be fundamentally prevented. Since the present disclosure may densely dispose the needles at intervals in a limited space area without short-circuiting, the manufacturing yield may be increased during the manufacturing process of the needle unit, and there is an effect of further improving the safety and therapeutic effect of the device.

Each of the needles included in the first needle group 211 and the second needle group 221 is not limited to a structure in which the cross-section is circular, and may be configured in various shapes such as triangles, squares, polygons, or a combination of these shapes.

In addition, each of the needles included in the first needle group 211 and the second needle group 221 may be configured to have a hollow part (not shown) in the center part so that a drug required for treatment may be injected.

A spacer 230 may be configured between the first circuit board 210 and the second circuit board 220 to prevent a short circuit.

In addition, a needle circuit board 240 may be configured in the needle unit 200 to be simultaneously connected to the first circuit board 210 and the second circuit board 220 so as to supply RF high-frequency energy to the first needle group 211 and the second needle group 221. A bracket 250 may be configured between the second circuit board 220 and the needle circuit board 240, and the needle circuit board 240 may be coupled to and fixed to the bracket. A shaft contact member 270 may be configured between the second circuit board 220 and the bracket 250. The shaft contact member 270 may contact with the end part of the shaft 302 that has come down by penetrating through a hole 241 formed in the center of the needle circuit board 240 and a hole 251 formed in the center of the bracket 250.

The upper side of the needle circuit board 240 may be provided with elastic springs 281, 282 having one end supported by the needle circuit board 240 and the other end fitted to needle electrode rods 291, 292 and supported by protrusion steps 291a, 292a of the needle electrode rods, respectively.

The upper side of the needle circuit board 240 may be provided with a connection terminal (not shown) that connects the circuits to each other by connecting the connection pin 428 of the main circuit board of the body block 400 when the needle unit 200 is coupled to the body part 100. The connection terminal configured on the needle circuit board 240 may be configured in the form of a copper foil surface to facilitate connection and disconnection of the connection pin 428 of the main circuit board.

The lower ends 291b, 292b of the needle electrode rods into which the elastic springs are fitted may be connected to the first circuit board 210 and the second circuit board 220 by penetrating the holes 242, 243 formed on both sides of the needle circuit board 240 and the holes 252, 253 formed on both sides of the bracket 250.

By configuring the needle electrode rods 291, 292 as described above, RF high-frequency energy and/or electric energy may be provided from the needle circuit board 240 to the first circuit board 210 and the second circuit board 220. In other words, the lower ends 291b, 292b of the needle electrode rods are circuitally connected so as to be able to supply electric energy to the first circuit board 210 and the second circuit board 220, and elastic springs 281, 282 are connected to the copper surfaces formed in the holes 242, 243 formed on both sides of the needle circuit board 240, and the springs 281, 282 are connected to the protrusion steps 291a, 292a of the electrode rods 291, 292 so as to be electrically connected to the needle circuit board 240 at all times. The needle electrode rods 291, 292 serve as movable terminals connected to the holes 242, 243 formed on both sides of the needle circuit board 240, so as to be electrically connected to the circuit of the needle circuit board 240 at all times.

Meanwhile, although the embodiments of the present disclosure mainly exemplify cases in which RF energy and/or electric energy is supplied through needles, depending on the embodiment, the skin treatment device 1000 according to the present disclosure may be used for purposes such as penetrating the skin or only providing stimulation from the outside of the skin without supplying such RF energy and/or electric energy. Accordingly, in the former case, adjacent needles may be disposed to have different polarities, but in the latter case, the needles may have no polarity or may have a single polarity.

Since the needle electrode rods 291, 292 are supported by elastic springs 281, 282 on the needle circuit board 240, they act to pull the combined assembly including the shaft contact member 270, the second circuit board 220, the spacer 230, and the first circuit board 210 toward the bracket 250, and when the driving motor 301 is driven to move the shaft 302 downward, the shaft contact member 270 is pushed so that the combined assembly including the first circuit board 210, the shaft contact member 270, and the like, may descend in the direction of travel, i.e., toward the skin, based on the bracket 250.

The combined assembly including the shaft contact member 270, the second circuit board 220, the spacer 230 and the first circuit board 210, and the bracket 250, the needle circuit board 240, and the needle electrode rods 291, 292 may be built into a tip case 297.

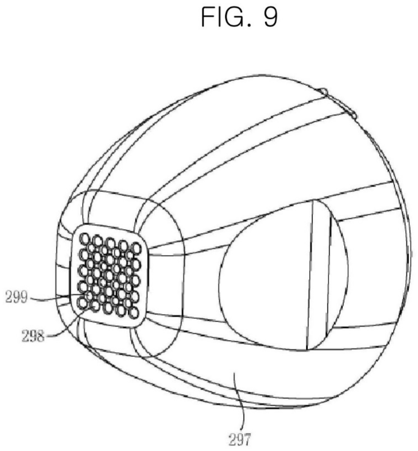

The tip case 297 may be formed with needle penetration holes 298, 299 through which the end parts of the first needle group 211 and the second needle group 221 pass through simultaneously. In addition, a fastening hole 296 for fastening with the body part 100 may be included. As shown in FIG. 9, the needle penetration hole 298 may be configured such that the first needle group 211 passes through, and the needle penetration hole 299 may be configured such that the second needle group 221 passes through. The length of the first needle group 211 and the second needle group 221 protruding outward from the needle penetration holes 298, 299 is determined according to the target depth of the wound caused by the needles, but may be 500 to 2,000 µm when targeting the dermis layer of the skin. In addition, as described above, the end points of the lengths of the first needle group 211 and the second needle group 221 protruding outward from the needle penetration holes 298, 299 may be formed to be the same.

Meanwhile, as described above, the skin treatment device 1000 provided with a needle group of the present disclosure may perform vibration during the treatment.

For example, the practitioner may generate a treatment start signal (e.g., power on) while the end part is in contact with the treatment area of the skin. Then, the vibration motor 425 is driven to generate a massage effect by the end part of the tip case 297 in contact with the skin, so that the stiffness of the skin may be relieved. When the driving motor 301 is driven to lower the shaft 302 at a certain interval in a state where the vibration is added or the vibration motor 425 is turned off after the massage, the end part of the shaft 302 pushes the shaft contact member 270 so that the first circuit board 210 and the second circuit board 220 are lowered simultaneously, so that the first needle group 211 and the second needle group 221 pierce the skin and a wound may be formed.

In this state, a treatment may be performed on the skin by irradiating RF high-frequency energy to the wound part through the first needle group 211 and the second needle group 221.

The above description of the present disclosure is for illustrative purposes, and those skilled in the art to which the present disclosure pertains will understand that the present disclosure may be easily modified into other specific forms without changing the technical idea or essential features of the present disclosure. Therefore, the embodiments described above should be understood in all respects as illustrative and not restrictive. For example, each component described as unitary may be implemented in a distributed manner, and similarly, components described as distributed may also be implemented in a combined form. The scope of the present disclosure is indicated by the appended claims rather than the detailed description above, and all changes or modified forms derived from the meaning and scope of the claims and their equivalent concepts should be construed as being included in the scope of the present disclosure.

### Industrial Applicability

The skin treatment device provided with a needle group of the present disclosure may be used for skin beauty, skin disease, local fat removal, and the like.

### Explanation of Symbols

100 - Body part
200 - Needle unit
210 - First circuit board
211 - First needle group
211a, 221a - Needle fixing part
212 - Perforation part
220 - Second circuit board
221 - Second needle group
230 - Spacer
240 - Needle circuit board
241, 242, 243, 251, 252, 253 - Hole
250 - Bracket
281, 282 - Elastic spring
291, 292 - Needle electrode rod
296 - Fastening hole
297 - Tip case
298, 299 - Needle penetration hole
300 - Motor block
301 - Driving motor
302 - Shaft
400 - Body block
401 - Shaft support
402 - Front support part
403 - Main circuit board support part
410 - Main circuit board
420 - LED window
425 - Vibration motor
428 - Connection pin
501,502 - Body case
520 - Cable
1000 - Skin treatment device

## Claims

1. A skin treatment device provided with a needle group, the skin treatment device comprising:
a needle group comprising a plurality of needles;
a first circuit board in which a first needle group, which is a part of the needle group, is fixed and a perforation part is formed; and
a second circuit board in which a second needle group, which is the remaining part of the needle group, is fixed,
wherein the second needle group is formed to protrude outward through the perforation part of the first circuit board.

2. The skin treatment device of claim 1, further comprising a tip case provided with a needle penetration hole through which the first needle group and the second needle group penetrate and protrude outward.

3. The skin treatment device of claim 1, wherein the number of needles included in the first needle group is greater than the number of needles included in the second needle group.

4. The skin treatment device of claim 1, wherein first needle fixing parts corresponding to the first needle group are arranged in a matrix having a plurality of rows and a plurality of columns, and
the perforation part is formed in a free space inside an area formed by the matrix that does not overlap with the first needle fixing parts.

5. The skin treatment device of claim 4, wherein the perforation part is formed inside a minimum rectangle that needle fixing parts included in the matrix are able to form.

6. The skin treatment device of claim 1, wherein needles included in the second needle group are formed to have a greater length than needles included in the first needle group.

7. The skin treatment device of claim 6, wherein the first needle group and the second needle group are formed such that respective end points are positioned at the same depth when maximally protruded outward.

8. The skin treatment device of claim 1, wherein at least one of the needles included in the needle group is provided with a hollow part to enable drug supply.

9. The skin treatment device of claim 1, further comprising a needle circuit board which is simultaneously connected to the first circuit board and the second circuit board so as to supply RF high-frequency energy to needles included in the first needle group and the second needle group, and a bracket configured between the second circuit board and the needle circuit board to support the needle circuit board.

10. The skin treatment device of claim 9, wherein an elastic spring having one end supported by the needle circuit board and a needle electrode rod fitted into the elastic spring are interposed such that circuit connections are made between the needle circuit board and the first circuit board and the second circuit board.

11. The skin treatment device of claim 10, wherein the skin treatment device comprises a main circuit board connected to the needle circuit board and a vibration motor, and further comprises a needle moving means configured to push at least the first circuit board and the second circuit board such that the needles included in the first needle group and the second needle group pass through the needle penetration hole of the tip case.

12. A skin treatment device provided with a needle group, the device comprising a plurality of needles, wherein the plurality of needles comprise:
a first needle group arranged in a rectangular matrix of m×n (where m is a natural number greater than or equal to 2 and the number of rows, and n is a natural number greater than or equal to 2 and the number of columns); and
a second needle group disposed inside a minimum rectangle formed by a needle disposed at (a, b) (where a is a row greater than or equal to 1 and less than or equal to (m-1), and b is a column greater than or equal to 1 and less than or equal to (n-1)), a needle disposed at (a, b+1), a needle disposed at (a+1, b), and a needle disposed at (a+1, b+1), among the first needle group.

13. A skin treatment device provided with a needle group, the device comprising a plurality of needles, wherein the plurality of needles comprise:
a first needle group arranged in a plurality of m rows (m is a natural number greater than or equal to 2), wherein among the m rows, n (n is a natural number greater than or equal to 2) needles are disposed in any one row a (a is a natural number greater than or equal to 1 and less than or equal to (m-1)) and (n-1) needles are disposed in row (a+1), wherein the needles disposed in the row (a+1) are disposed so as to be positioned between the needles of the row a in an x-axis direction; and
a second needle group disposed inside (e.g., at the center of) a minimum triangle formed by a needle disposed at (a, b) (a is a natural number greater than or equal to 1 and less than or equal to (m-1), and b is a natural number greater than or equal to 1 and less than or equal to (n-1)), a needle disposed at (a, b+1), and a needle disposed at (a+1, b), among the first needle group.

14. A method of driving a skin treatment device provided with a needle group, the method comprising:
receiving, by the skin treatment device provided with the needle group, a treatment start signal; and
moving, by the skin treatment device provided with the needle group, a first circuit board in which a first needle group, which is a part of a needle group comprising a plurality of needles, is fixed and a perforation part is formed, and a second circuit board in which a second needle group, which is the remaining part of the needle group, is fixed, in response to the treatment start signal,
wherein the second needle group is formed to protrude outward through the perforation part of the first circuit board.
